# EUROPEAN PATENT APPLICATION

(11) **EP 1 088 872 A2**
(43) Date of publication of application: **04.04.2001**
(21) Application number: 00307775.7
(22) Date of filing: 08.09.2000
(51) Int. Cl.: C09K 3/00

(54) **Near infrared absorbing compound**

(30) Priority: 28.09.1999 JP 27475899
(71) Applicant: NISSHINBO INDUSTRIES, INC., Chuo-ku, Tokyo (JP)
(72) Inventor: Hasegawa, Shun, Chiba-shi, Chiba (JP); Masuda, Gen, Chiba-shi, Chiba (JP)
(74) Representative: Tombling, Adrian George

(57) **Abstract**

By forming a copper salt of phytic acid at a molar ratio of copper ion (Cu²⁺) to phytic acid of 2:1 or higher, there is provided a compound absorbing near infrared rays, but which does not absorb visible light, and shows extremely good water solubility.

## Description

The present invention relates to a novel substance that efficiently absorbs light in the near infrared wavelength range.

Materials that absorb light in the near infrared wavelength range are utilized as heat ray absorbers or optical filters.

Various materials including those mentioned below have been examined so far.

For example, metal iron oxides such as ferrous oxide are known to absorb heat rays. However, since their absorption coefficient is low, and they suffer from a problem of poor solubility in solvents, they are not practically useful. Further, copper carboxylates as organic copper salts such as copper benzoate, copper acetate and copper naphthenate are also known to absorb heat rays. However, they have a maximum absorption wavelength of around 600-750 nm, and they only show weak absorption in the near infrared range.

It has also been reported that a water-based solution of ferrous sulfate or ferrous ammonium sulfate absorbs light of near infrared wavelength (Japanese Patent Laid-open (Kokai) Nos. 63-116625/1998, Japanese Patent Laid-open (Kokai) Nos 63-116626/1998). However, if a solution of these sulfates is left for a while, they react with the water to generate deposits or precipitates. Therefore, they suffer from a problem of troublesome handling, i.e., a suitable amount of sulfuric acid must be added to the solution in order to avoid such deposition or precipitation.

It is noted that phthalocyanine and napthalocyanine are known as copper-containing organic compounds absorbing near infrared rays. However, both of these show absorption in the visible light range. Further, they have a maximum absorption wavelength of 800 nm or shorter with a sharp peak, and hence they do not show absorption over a broad wavelength range.

Furthermore, there has been proposed an optical filter for absorbing light in the near infrared range, characterized in that it comprises a copolymer obtained by copolymerizing a monomer with a specific structure having a phosphoric acid group and a monomer copolymerizable with the former monomer, and a metal salt mainly consisting of a copper salt (Japanese Patent Laid-open (Kokai) No. 6-118228/1994). There have also been proposed a near infrared absorbing resin composition comprising a resin obtained by polymerizing a monomer having an unsaturated double bond, a phosphorus atom-containing compound with a specific structure and copper hydroxide, and a near infrared absorbing resin composition comprising a phosphorus atom-containing compound with a specific structure (Japanese Patent Laid-open (Kokai) Nos. 10-152598/1998 and 10-153964/1998). Although these compositions absorb light in the near infrared wavelength range, they suffer from the problem of low water-solubility.

An object of the present invention is to provide a compound absorbing near infrared rays, which does not substantially absorb light in the visible light wavelength range, but strongly absorbs light in the near infrared wavelength range, and shows extremely good water solubility.

As a result of assiduous studies we have found that particular copper salts of naturally-occurring phytic acid strongly absorb light in the near infrared wavelength range.

According to one aspect on we provide a copper salt of phytic acid, which is formed from cupric ion (Cu²⁺) and phytic acid at a molar ration of cupric ion to phytic acid of 2:1 or higher.

This copper salt of phytic acid may be obtained by reacting an inorganic copper salt or an organic copper salt with phytic acid.

Preferably the organic copper salt is a copper salt of a C2-C8 carboxylic acid.

We also provide a near infrared absorber comprising a copper salt of phytic acid as defined hereinabove.

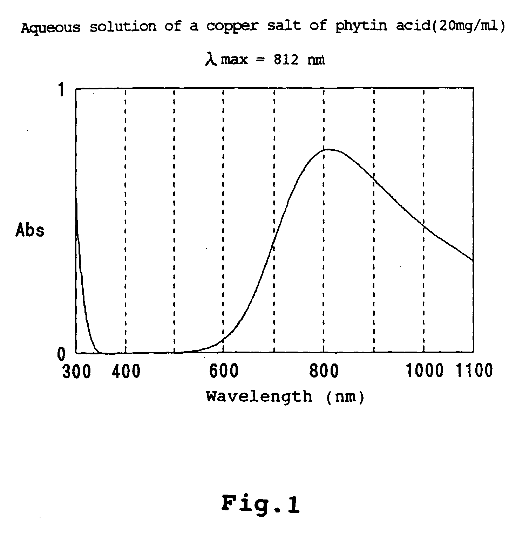

Embodiments of the invention will not be described by way of example and with reference to Fig 1, which shows an absorption spectrum of a compound of the present invention.

### 〈1〉 Copper salt of phytic acid.

Phytic acid exists in nature, and it is a compound corresponding to myoinositol of which six hydroxyl groups have been substituted with phosphoric acid groups (-P(O)(OH)₂). In the present invention, phytic acid may include compounds that have been partially dephosphorylated, and the term "phytic acid" is used to encompass such substances.

Commercially available phytic acids may be used. For example, 50% phytic acid (Tokyo Kasei Co., Ltd.) can be used, which also contains phytic acid partially dephosphorylated.

The copper salt of phytic acid is formed by substitution of one mole of cupric ions (Cu²⁺) for the two hydrogen atoms of phosphoric acid groups in 1 mole of phytic acid. In the present invention, a copper salt of phytic acid in which cupric ions (Cu²⁺) and phytic acid form a salt at a molar ratio of 2:1 or higher means a salt formed with 1 mole of phytic acid and 2 moles or more of copper ions (Cu²⁺). That is, when the copper salt is formed with 1 mole of phytic acid and 2 moles of copper ions (Cu²⁺), four hydrogen atoms of the phosphoric acid groups in the phytic acid molecule are substituted with two copper ions (Cu²⁺).

The molar ration of phytic acid:copper ions (Cu²⁺) may be 1:6 at most.

The copper salt of phytic acid of the present invention can be obtained by reacting an inorganic copper salt or an organic copper salt with the aforementioned phytic acid.

As the inorganic copper salt used for the reaction, copper sulfate, copper chloride, copper carbonate can be mentioned as examples. While the amount of the inorganic copper salt varies depending on the type of the inorganic copper salt to be used, it is preferably used in an amount of 0.5-6.0 molar equivalents, more preferably 1.5-2.5 molar equivalents on the phosphoric acid groups of phytic acid.

When an organic copper salt is used, copper salts of carboxylic acids for example copper acetate, copper formate, copper stearate, copper tartrate, copper citrate and copper benzoate can be used, and copper acetate is preferred.

While the amount of the organic copper salt varies depending on the type of the organic copper salt to be used, it is preferably used in an amount of 0.5-6.0 molar equivalents, more preferably 1.5-2.5 molar equivalents on the phosphoric acid groups of phytic acid.

The reaction temperature varies depending on the kind of reactants and so forth. However, it is generally 10-90°C, preferably 20-30°C.

The reaction time, i.e. the time required for full dissolution of the reactants, varies depending on the kinds of the reactants and so forth, but it is usually around 0.5 to 4 hours.

After the reaction has been completed the reaction mixture can be added dropwise to an organic solvent that is miscible with water, and the resulting deposit of the copper salt of phytic acid collected by, for example, filtration.

The organic solvent that is miscible with water may be a poor solvent that brings about deposition of the copper salt of phytic acid after completion of the reaction. Examples thereof include lower alcohols such as methanol, ethanol, n-propanol and isopropanol, acetonitrile, acetone, dimethylformamide, dimethyl sulfoxide, tetrahydrofuran, and mixtures of these.

The solid obtained as described above may be, for example, washed with a solvent such as an alcohol, as required.

### 〈2〉 Near infrared absorber

The present invention also provides a near infrared absorber comprising the aforementioned copper salt of phytic acid.

Since the copper salt of phytic acid of the present invention also has ability to absorb light in the ultraviolet wavelength range, it can also serve as an ultraviolet absorber.

The near infrared absorber of the present invention can be made as, for example, a film that can be obtained by adding a suitable amount of the copper salt of phytic acid of the present invention to a synthetic resin such as vinyl resin. This film is preferably a transparent film.

Further, since the copper salt of phytic acid of the present invention shows extremely high solubility in water, the copper salt of phytic acid can also be used in the form of an aqueous solution. In this case, the near infrared absorber may be, for example, a filter that comprises an aqueous solution of the copper salt of phytic acid which is sealed between two parallel transparent plates, which may be of a synthetic resin such as polycarbonate, acrylic resin or vinyl chloride, or alternatively of plate glass.

By adhering the aforementioned film or filter to window glass of buildings, it becomes possible to shield lights of near infrared wavelength range. Since the compound of the present invention does not shield visible light unlike curtains or blinds, it can shield light of near infrared wavelength range without reducing indoor brightness.

Furthermore, if the film or filter is used as a film or filter in plant cultivation, good growing ability of the plants can be maintained, since the film or filter shields heat rays and ultraviolet rays harmful to plant growth, but does not shield the visible light required for plant growth.

The film and filter can also be utilized as an optical filter such as a filter for light measurement that adjusts the characteristics of photodiodes.

The present invention will be more specifically explained with reference to the following non-limiting example.

### Example

Into 13.7g of 50% phytic acid solution (Tokyo Kasei Co., Ltd), 4.15g of copper acetate monohydrate (Wako Pure Chemical Industries, Ltd) was added and fully dissolved by stirring. This solution was added little by little dropwise to a vigorously-stirred mixture of 400 ml of isopropanol and 50 ml of methanol. After deposition of crystals, the crystals were filtered off under reduced pressure by using a Kiriyama funnel, and washed several times with isopropanol to obtain a solid showing slight viscosity. The solid was wetted with methanol, and pulverized to a fine powder with a spatula.

To the powder was added methanol again, and the mixture was stirred for 30 minutes to wash it, and then filtered again using a Kiriyama funnel and dried in vacuo to obtain 6.60g of near infrared absorbing composition as a blue powder.

The ratio of copper to phosphoric acid groups in this powder was analyzed, and it was found to be 1:2.45.

This powder was dissolved in ion-exchanged water at a concentration of 20 mg/ml, and the absorption spectrum of the solution was determined. The result is shown in Fig. 1.

The compound of the present invention, of which the absorption spectrum is shown in Fig. 1, shows substantially no absorption in the region of 400-600 nm, which corresponds to visible light, but shows absorption over a wide range from 750-1100 nm.

Thus, according to the present invention, there can be provided a near infrared absorbing compound that hardly absorbs visible light, but strongly broadly absorbs lights of near infrared wavelength range.

Further, the compound of the present invention is highly water-soluble. Therefore, it can be utilized for aqueous filter devices by being dissolved in water at a high concentration to provide such devices absorbing near infrared light but transmitting well visible light.

Furthermore, the compounds of the present invention can be produced at low cost, since they are obtained from raw materials abundantly present in nature.

## Claims

1. A copper salt of phytic acid, which is formed from cupric ions (Cu²⁺) and phytic acid at a molar ration of cupric ion to phytic acid of 2:1 or higher.

2. The copper salt of phytic acid according to claim 1, which is obtainable by reacting an inorganic cupric salt or an organic cupric salt with phytic acid.

3. The copper salt of phytic acid according to claim 1 or 2, wherein the organic cupric salt is a copper cupric salt of a C2-C8 carboxylic acid.

4. A near infrared and/or ultraviolet absorber comprising a copper salt of phytic acid according to any of claims 1 to 3.
